# EUROPÄISCHE PATENTSCHRIFT

(11) **EP 4 373 469 B1**
(45) Veröffentlichungstag und Bekanntmachung des Hinweises auf die Patenterteilung: **02.09.2026**
(21) Anmeldenummer: 22750768.8
(22) Anmeldetag: 11.07.2022
(51) Int. Cl.: A61K 9/00, A61K 9/14, A61K 31/155, A61M 5/00, A61M 37/00, A61M 5/32

(54) **MIKRONADELARRAY MIT ANTISEPTIKA**
MICRONEEDLE ARRAY WITH ANTISEPTICS
RÉSEAU DE MICRO-AIGUILLES AVEC D'ANTISEPTIQUES

(30) Priorität: 22.07.2021 DE 102021118997
(43) Veröffentlichungstag der Anmeldung: 29.05.2024
(73) Patentinhaber: LTS Lohmann Therapie-Systeme AG, 56626 Andernach (DE)
(72) Erfinder: WINTERBERG, Markus, 5359 Rheinbach (DE)
(74) Vertreter: dompatent
(86) Internationale Anmeldenummer: PCT/EP2022/069218
(87) Internationale Veröffentlichungsnummer: WO 2023/001607

(56) Entgegenhaltungen:
- EP-A2- 0 328 421
- WO-A1-2015/044669
- WO-A1-2019/202170
- WO-A1-2020/250210
- WO-A2-2008/130587
- CN-A- 112 472 660
- ROGALSKY SERGIY ET AL: "New promising antimicrobial material based on thermoplastic polyurethane modified with polymeric biocide polyhexamethylene guanidine hydrochloride", MATERIALS CHEMISTRY AND PHYSICS, ELSEVIER SA, SWITZERLAND, TAIWAN, REPUBLIC OF CHINA, vol. 267, 3 May 2021 (2021-05-03), XP086615212, ISSN: 0254-0584, [retrieved on 20210503], DOI: 10.1016/J.MATCHEMPHYS.2021.124682
- KIRKBY MELISSA ET AL: "Microneedle Mediated Transdermal Delivery of Protein, Peptide and Antibody Based Therapeutics: Current Status and Future Considerations", PHARMACEUTICAL RESEARCH, SPRINGER US, NEW YORK, vol. 37, no. 6, 2 June 2020 (2020-06-02), XP037200675, ISSN: 0724-8741, [retrieved on 20200602], DOI: 10.1007/S11095-020-02844-6

## Beschreibung

Die vorliegende Erfindung betrifft ein Mikronadelarray zur Verwendung in der intradermalen Applikation von Wirkstoffen, das eine Vielzahl von Mikronadeln auf einem Träger umfasst, die aus einer Formulierung gebildet sind, die ein biologisch abbaubares Polymer, einen Wirkstoff und ein Antiseptikum enthält, wobei das Antiseptikum und der Wirkstoff in die Polymermatrix der Mikronadeln integriert sind, und es sich bei dem Wirkstoff um einen Impfstoff handelt.

Die Haut als funktionell vielseitigstes Organ des menschlichen und tierischen Organismus besteht aus mehreren Schichten und übernimmt neben ihrer Aufgabe als Hüllorgan wesentliche Funktionen im Bereich des Stoffwechsels, der Wärmeregulation und der Immunantwort. Die äußerste Hautschicht, die sogenannte Hornhaut oder *Stratum Corneum,* weist bekannte Sperreigenschaften auf, um ein Eindringen von Fremdsubstanzen in den Körper und ein Austreten von Eigensubstanzen aus dem Körper zu verhindern. Das *Stratum Corneum,* das eine komplexe Struktur aus kompaktierten keratotischen Zellresten mit einer Dicke von ungefähr 10 bis 30 Mikrometern ist, bildet eine wasserdichte Membran zum Schutz des Körpers. Die natürliche Undurchlässigkeit des *Stratum Corneum* verhindert das Verabreichen der meisten pharmazeutischen Mittel und anderer Substanzen durch die Haut im Rahmen einer transdermalen Applikation.

Mikronadelsysteme (MNS), die aus einem Mikronadelarray (MNA) und gegebenenfalls weiteren Komponenten bestehen, haben sich in letzter Zeit als Alternative zur klassischen transdermalen Applikation, wie transdermale Pflaster, von pharmazeutischen und kosmetischen Mitteln etabliert. Während transdermale Pflaster auf die Haut geklebt werden und der freigesetzte Wirkstoff über die Haut resorbiert wird, werden die Mikronadeln, die teilweise auch als Hautdurchdringungselemente bezeichnet werden, mittels einer Druckkraft des Arrays gegen die Applikationsstelle auf die Haut gepresst, um das *Stratum Corneum* zu durchdringen und auf diese Weise einen Fluidkanal herzustellen, so dass ein Wirkstoff intradermal appliziert werden kann. Solche Mikronadelarrays in Mikronadelsystemen, deren Herstellung sowie deren Verwendung zur Applikation einer Reihe von Wirkstoffen sind im Stand der Technik bekannt.

Eine Übersicht über die aktuellen Entwicklungen auf dem Gebiet der Mikronadeln bietet der Artikel *"*Mikroneedles as an alternative technology for transdermal drug delivery systems: a patent review" von M.L. Barreto Queiroz et al, erschienen in EXPERT OPINION ON THERAPEUTIC PATENTS 2020, Vol. 30, Nr. 6, S. 433-452. Hier werden unter anderem die verschiedenen Systeme vorgestellt, wie feste, wiederentfernbare Mikronadeln, beschichtete Mikronadeln, sich auflösende Mikronadeln, hohle Mikronadeln und Mikronadeln, die ein Hydrogel bilden, wobei insbesondere das Konzept der sich auflösenden Mikronadeln in letzter Zeit in den Fokus der Aufmerksamkeit gerückt ist.

So offenbart WO 2019/115815 ein Mikronadelarray zur Verwendung in der intradermalen Applikation eines Wirkstoffs in Form eines Salzes umfassend eine Vielzahl von Mikronadeln an einem Träger, wobei die Mikronadeln eine Formulierung umfassen, die mindestens einen Wirkstoff in Form eines Salzes und mindestens ein biologisch abbaubares Polymer enthält.

A.D. Permana et al beschreiben in ihrem Artikel "Solid lipid nanoparticle-based dissolving microneedles; A promising intradermal lymph targeting drug delivery system with potential for enhanced treatment of lymphatic filariasis", erschienen in Journal of Controlled Release 316 (2019) 34-52, die Verwendung von sich auflösenden Mikronadeln zur Behandlung von lymphatischer Filariose.

WO 2019/092257 beschreibt ein Mikronadelarray umfassend eine Formulierung aus Polyvinylpyrrolidon und HBsAg zur Verwendung in der intradermalen Applikation zur Hepatits-Impfung.

WO 2018/224559 bezeichnet ein Mikronadelarray umfassend eine Formulierung aus Polyvinylpyrrolidon und mindestens einem Glukagon-ähnlichen-Peptid Analoga zur Verwendung in der intradermalen Applikation zur kontrollierten Freisetzung.

WO 2019/202170 betrifft ein Mikronadelarray umfassend eine vollständig lösbare Formulierung zur Verwendung in der intradermalen Applikation von Interferon, wobei Polyvinylpyrrolidon Hauptbestandteil der Formulierung ist.

Insbesondere bei Mikronadeln, die zur Verabreichung von Protein- und Nukleinsäurewirkstoffen vorgesehen sind, stellt sich das Problem der Bereitstellung eines sterilen Produkts, da die derzeit zugelassenen Sterilisationsverfahren, wie zum Beispiel die Gammasterilisation, zu einer Zersetzung der zu verabreichenden Wirkstoffe führen. Um sterile Produkte zu erhalten ist daher eine teure und aufwendige Sterilfertigung notwendig.

Um diesem Problem zu begegnen, schlägt US 2017/0028184 ein Mikronadelsystem mit einer *in situ* Anode und Kathode vor, das dadurch erreicht wird, dass die Mikronadeln aus einem Material gefertigt sind, das ein elektrisches Potential aufweist. Den zur Herstellung der Mikronadeln verwendeten Metallen werden antiseptische Eigenschaften zugeschrieben.

EP 3 669 929 beschreibt ein Mikronadelarray zum Auffüllen von Lippen, wobei die Mikronadeln neben einem wasserlöslichen Polymer ein Antiseptikum enthalten können.

US 2019/0358441 beschreibt ein Mikronadelpflaster mit einem Substrat, einer Mikronadelmatrix, die mehr als eine Mikronadel enthält, wobei jede Mikronadel eine Basis, ein kegelförmiges spitzes Ende, das mit einem Gemisch aus einem biolöslichen Trägermaterial mit einer aktiven Komponente gefüllt ist, und eine Vielzahl breiterer kegelförmiger Verzweigungen zwischen dem spitzen Ende und der Basis aufweist, die sich geometrisch miteinander kreuzen und mit einem biolöslichen Material gefüllt sind; einem Basisfilm, der die Basen der Mikronadeln in der Mikronadelmatrix mit einer inneren Oberfläche verbindet und mit einer äußeren Oberfläche an dem Substrat befestigt ist; wobei das scharfe Ende der Mikronadel oben auf den Verzweigungen der Mikronadel angeordnet ist, und wobei das Substrat und der Basisfilm aus flexiblen Materialien hergestellt sind.

WO 2011/127149 offenbart ein Verfahren zur kontrollierten Freisetzung einer wirksamen Menge mindestens eines oder mehrerer bioaktiver oder pharmazeutisch aktiver Wirkstoffe in einem Subjekt, umfassend die Verabreichung eines Kombinations-Biomaterials an ein Subjekt, wobei das Kombinations-Biomaterial ein Kombinations-Biomaterial-Substrat und ein abbaubares Polymer umfasst, wobei das abbaubare Polymer einen oder mehrere bioaktive oder pharmazeutisch aktive Wirkstoffe umfasst, die durch das abbaubare Polymer eingekapselt sind, wobei der eine oder die mehreren bioaktiven Wirkstoffe oder pharmazeutisch aktiven Wirkstoffe dem Subjekt über einen Zeitraum von mehr als einer Woche zugeführt werden.

WO 2021/077119 stellt quaternäre Organosilizium-Ammonium-Verbindungen und deren Zusammensetzungen für die topische medizinische Therapie bei Menschen und Tieren zur Verfügung.

Trotz umfangreicher Bemühungen im Stand der Technik besteht bei der Verwendung von Mikronadeln weiterhin das Restrisiko einer Infektion durch das Einbringen von Pathogenen in die Haut. Daher besteht die Aufgabe der vorliegenden Erfindung darin, ein Mikronadelarray zur Verfügung zu stellen, mit dem dieses Risiko weiter gesenkt werden kann und das zur Applikation einer Vielzahl von Wirkstoffen geeignet ist.

Im Rahmen der vorliegenden Erfindung wurde überraschend gefunden, dass diese Aufgabe durch ein Mikronadelarray gelöst wird, bei dem die Mikronadeln aus einer Co-Formulierung gebildet sind, die ein biologisch abbaubares Polymer und ein Antiseptikum enthält, wobei das Antiseptikum und der Wirkstoff in die Polymermatrix der Mikronadeln integriert sind, und es sich bei dem Wirkstoff um einen Impfstoff handelt.

Daher ist ein erster Gegenstand der vorliegenden Erfindung ein Mikronadelarray zur Verwendung in der intradermalen Applikation, umfassend eine Vielzahl von Mikronadeln auf einem Träger, wobei die Mikronadeln aus einer Co-Formulierung gebildet sind, die ein biologisch abbaubares Polymer, einen Wirkstoff und ein Antiseptikum mit mindestens einer Guanid-/und oder Biguanidgruppe enthält, wobei das Antiseptikum und der Wirkstoff in die Polymermatrix der Mikronadeln integriert sind, und es sich bei dem Wirkstoff um einen Impfstoff handelt.

Das erfindungsgemäße Mikronadelarray weist den Vorteil auf, dass auf eine aufwendige sterile Herstellung der Mikronadeln verzichtet werden kann. Vielmehr weisen die erfindungsgemäßen Mikronadelarrays trotz Herstellung in einem "low bioburden" Umfeld die nötige Sterilität auf, um ein Infektionsrisiko weiter zu minimieren.

Im Gegensatz zu im Stand der Technik vorgeschlagenen Lösungen, das Antiseptikum in Hohlräumen der Mikronadeln unterzubringen, ist das Antiseptikum in die Polymermatrix der Mikronadeln eingebunden und/oder eingebettet und unmittelbar verfügbar. Darüber hinaus bietet die erfindungsgemäße Lösung den Vorteil, dass in den Mikronadeln gegebenenfalls vorgesehene Hohlräume für die Aufnahme weiterer Wirkstoffe verwendet werden können und nicht durch das Antiseptikum blockiert werden.

Das Antiseptikum ist ausgewählt aus der Gruppe der Verbindungen aus der Klasse der Guanide und Biguanide, die neben einem vorteilhaften Einbau in die Polymermatrix des biologisch abbaubaren Polymers auch eine gute Verträglichkeit mit einer Vielzahl von gängigen Wirkstoffen zeigen.

Im Rahmen der vorliegenden Erfindung handelt es sich um ein Antiseptikum, das eine Guanid-Gruppe aufweist, wenn die entsprechende Verbindung in ihrer chemischen Struktur das folgende Strukturelement aufweist:

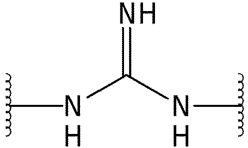

Unter einem Antiseptikum, das eine Biguanidgruppe aufweist, sind solche chemischen Verbindungen zu verstehen, die das folgende Strukturelement aufweisen:

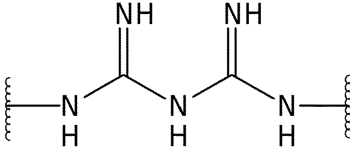

Guanide und Biguanide sind als antimikrobielle Wirkstoffe im Stand der Technik bekannt. Die Guanide und Biguanide können als Monomere oder bevorzugt als Polymere, also als Polyguanid und/oder Polybiguanid eingesetzt werden. Die Guanide und/oder Biguanide liegen in einer bevorzugten Ausführungsform als wasserlösliche physiologisch akzeptable Salze vor. Besonders bevorzugt liegen die Guanide und Biguanide als Hydrohalogenid, beispielsweise Hydrochlorid oder Hydrobromid vor.

Besonders gute Ergebnisse bezüglich der Verträglichkeit mit anderen Wirkstoffen und einer antiseptischen Wirkung wurden beobachtet, wenn als Antiseptikum ein Polybiguanid eingesetzt wird. Daher ist eine Ausführungsform der vorliegenden Erfindung bevorzugt, in der es sich bei dem Antiseptikum um ein Polybiguanid, vorzugsweise um Chlorhexidin und/oder Polyhexamethylenbiguanid (PHMB), insbesondere Polyhexamethylenbiguanid (PHMB) handelt.

Die erfindungsgemäßen Mikronadelarrays sind insbesondere für die transdermale Applikationen von kosmetischen und pharmazeutischen Wirkstoffen, insbesondere pharmazeutischen Wirkstoffen, vorgesehen. Sie bieten dabei den Vorteil, dass der Wirkstoff zusammen mit dem Antiseptikum und dem biologisch abbaubaren Polymer als homogene Formulierung vorliegt, aus der die Mikronadeln hergestellt sind. Der Wirkstoff und das Antiseptikum werden durch Auflösen des Polymers freigesetzt und stehen somit direkt an der Applikationsstelle zur Verfügung.

Daher ist der Wirkstoff, insbesondere ein Arzneimittel, in den Mikronadeln oder in die Matrix oder Formulierung des Mikroarrays integriert bzw. eingearbeitet. Weiterhin kann mindestens ein Wirkstoff, insbesondere Arzneimittel, auf den Mikronadeln oder auf dem Träger aufgebracht sein. Bevorzugt ist jedoch, dass der Wirkstoff, insbesondere Arzneimittel, Bestandteil der Mikronadeln ist und hierzu in die Mikronadeln eingebracht oder eingearbeitet ist, insbesondere in der Spitze der Mikronadeln.

Erfindungsgemäß eingesetzte Wirkstoffe sind insbesondere Arzneimittel wie sie in der EU-Richtlinie 2001/83/EG (Gemeinschaftskodex für Humanarzneimittel) definiert sind.

Im Rahmen der vorliegenden Erfindung hat sich gezeigt, dass die erfindungsgemäßen Mikronadelarrays für die Applikation einer Vielzahl von Wirkstoffen geeignet sind. So konnte bei ersten Versuchen keine Abnahme der Wirkstoffaktivität beobachtet werden. Vorzugsweise handelt es sich bei dem Wirkstoff um einen pharmazeutischen Wirkstoff. Vorzugsweise ist der Wirkstoff ausgewählt aus der Gruppe bestehend aus Nukleinsäuren, Peptiden und Proteinen.

Insbesondere handelt es sich bei dem Wirkstoff um einen Impfstoff.

In einer weiterhin bevorzugten Ausführungsform handelt es sich bei dem Wirkstoff um eine Nukleinsäure, insbesondere mRNA und silencer mRNA.

In einer bevorzugten Ausführungsform liegt der Wirkstoff in Form von Lipid-Nanopartikeln vor. Der Wirkstoff kann einzeln oder in Kombination mehrerer Wirkstoffe eingesetzt werden.

In einer bevorzugten Ausführungsform weisen die Mikronadeln einen mehrschichtigen Aufbau auf, wobei die Schichten jeweils aus einem biologisch abbaubaren Polymer gebildet sind. Vorzugsweise wird mindestens eine Schicht aus einer Formulierung gebildet, die ein biologisch abbaubares Polymer, einen Wirkstoff und ein Antiseptikum enthält. In einer besonders bevorzugten Ausführungsform bildet diese Schicht die äußere Schicht der Mikronadeln. In einer bevorzugten Ausführungsform wird mindestens eine der inneren Schichten aus einer Formulierung gebildet, die ein biologisch abbaubares Polymer und ein Antiseptikum enthält. Auf diese Weise kann eine sich über einen längeren Zeitraum erstreckende Abgabe des Antiseptikums erreicht werden.

In einer weiteren alternativ bevorzugten Ausführungsform wird die äußere Schicht der Mikronadeln aus einer Formulierung gebildet, die ein biologisch abbaubares Polymer und ein Antiseptikum enthält. Auf diese Weise kann das Antiseptikum bereits an der Applikationsstelle wirken, bevor der Wirkstoff freigesetzt wird.

Die Mikronadeln gemäß der vorliegenden Erfindung sind aus einer Formulierung hergestellt, die ein biologisch abbaubares Polymer enthält. Besonders bevorzugt bestehen die Mikronadeln aus einer Co-Formulierung aus einem biologisch abbaubaren Polymer, einem Wirkstoff und einem Antiseptikum. In der praktischen Anwendung haben sich insbesondere Mikronadelarrays als vorteilhaft erwiesen, die sich selbst auflösen. Dies sind insbesondere Mikronadelarrays, die nach der Applikation in der Haut verbleiben und sich unter Freisetzung des Wirkstoffs auflösen. Durch den Verbleib der Mikronadeln in der Haut kann eine kurze Applikationszeit erreicht werden, wodurch sich beispielsweise Irritationen der Hautoberfläche, die durch das Trägermaterial hervorgerufen werden können, vermeiden lassen. Weiterhin ermöglicht diese Methode eine unmittelbare und konstante Wirkstofffreisetzung. Damit die Mikronadeln geeignet sind, in der Haut zu verbleiben, müssen die zu ihrer Herstellung verwendeten Materialien zum einen toxikologisch unbedenklich und zum anderen selbstauflösend sein. Diese Kombination an Eigenschaften wird insbesondere dann erreicht, wenn die Mikronadeln aus bestimmten biologisch abbaubaren Polymeren bestehen.

Daher ist eine Ausführungsform bevorzugt, in der das biologisch abbaubare Polymer ausgewählt ist aus der Gruppe bestehend aus Polyvinylpyrrolidon, Polyvinylalkohole, Cellulose, Dextrane, Glycane, Glycosaminglycane, Hyaluronen, alpha-Hydroxysäuren, solche wie Milchsäure und/oder Glykolsäure, Polylactide, Polyglykolide, Polylactid-co-Glykolide, und Copolymere davon mit Polyethylenglykolen, Polyanhydriden, Poly(ortho)estern, Polyurethanen, Polybuttersäuren, Polyvaleriansäuren und Polylactid-co-Caprolactonen. In einer besonders bevorzugten Ausführungsformen ist das biologisch abbaubare Polymer ausgewählt aus Polyvinylpyrrolidonen und Dextranen.

Das Mikronadelarray kann eine Vielzahl von Mikronadeln aufweisen, um einen Stoff über die Haut oder in die Haut eines Patienten abzugeben, wobei das Mikronadelarray auf die Haut des Patienten aufgebracht wird. Jede der Mikronadeln des Mikronadelarrays weist vorzugsweise einen langgestreckten Schaft mit zwei Enden auf, das eine Ende des Schafts ist die Basis der Mikronadel, mit der die Mikronadel an dem flächigen Träger befestigt oder mit der die Mikronadel in den flächigen Träger integriert ist. Das der Basis gegenüberliegende Ende des Schafts ist vorzugsweise spitz zulaufend ausgestaltet, um ein möglichst leichtes Eindringen der Mikronadel in die Haut zu ermöglichen. Eine hohle Mikronadel kann mindestens einen Durchgang beziehungsweise Kanal oder mindestens eine Bohrung aufweisen, der/die sich von der Basis der Mikronadel bis zur Spitze der Mikronadel oder bis annähernd zur Spitze der Mikronadel erstreckt. Die Durchgänge weisen vorzugsweise einen runden Durchmesser auf.

Die Mikronadeln können einen Schaft mit einem runden Querschnitt oder einen nicht runden Querschnitt aufweisen, beispielsweise mit einem dreieckigen, viereckigen oder einem polygonalen Querschnitt. Der Schaft kann einen Durchgang oder mehrere Durchgänge aufweisen, die von der Nadelbasis bis zur Nadelspitze oder annähernd zur Nadelspitze verläuft/verlaufen. Die Mikronadeln können als (Wider-)Haken ausgebildet sein, wobei ein oder mehrere dieser Mikronadeln einen oder mehrere solcher Haken aufweist. Weiterhin können die Mikronadeln schraubenförmig gestaltet und drehbar angeordnet sein und dadurch beim Anwenden einer rotierenden Bewegung das Eindringen in die Haut erleichtern und eine Verankerung in der Haut bewirken, wie beispielsweise in DE 103 53 629 A1 beschrieben, insbesondere in der gewünschten Penetrationstiefe in der Epidermis.

Der Durchmesser einer Mikronadel beträgt üblicherweise zwischen 1 µm und 500 µm, vorzugsweise zwischen 10 µm und 100 µm. Der Durchmesser eines Durchgangs beträgt üblicherweise zwischen 3 µm und 80 µm und ist für das Durchtreten von vorzugsweise flüssigen Stoffen, Lösungen und Stoffzubereitungen geeignet. Die Länge einer Mikronadel beträgt üblicherweise zwischen 10 µm und 1.000 µm, insbesondere zwischen 100 µm und 500 µm.

Die Mikronadeln sind mit ihrer Basis an einem flächigen Träger befestigt oder in einen flächigen Träger integriert. Die Mikronadeln sind vorzugsweise im Wesentlichen senkrecht zur Fläche des Trägers stehend angeordnet. Die Mikronadeln können regelmäßig oder unregelmäßig angeordnet sein. Eine Anordnung von mehreren Mikronadeln kann Mikronadeln mit unterschiedlichen Querschnittformen, unterschiedlich dimensionierten Durchmessern und/oder unterschiedlichen Längen aufweisen. Die Anordnung aus mehreren Mikronadeln kann ausschließlich hohle Mikronadeln aufweisen. Die Anordnung kann sowohl massive Mikronadeln umfassen als auch teilmassive Komposite, wie beispielsweise mit Flüssigkeitseinschlüssen durchsetzte, massive Mikronadeln aufweisen.

Das Mikronadelarray kann einen flächigen Träger aufweisen, wobei der Träger im Wesentlichen eine scheibenförmige, plattenförmige oder folienförmige Grundform hat. Der Träger kann eine runde, ovale, dreieckige, viereckige oder polygonale Grundfläche haben. Der Träger kann aus unterschiedlichen Materialien hergestellt sein, beispielsweise aus einem Metall, einem keramischen Werkstoff, einem Halbleiter, einem organischen Material, einem Polymer oder einem Komposit. Als Materialien, die zur Herstellung des Trägers geeignet sind, können vorzugsweise Folien oder bahnförmige Materialien genannt werden, beispielsweise mikroporöse Membranen, vorzugsweise aus Polyethylen (PE) oder Polypropylen (PP), oder Diffusions-Membranen, vorzugsweise aus Ethylen-Vinylacetat-Copolymer (EVA) oder Polyurethan (PU). Geeignete Materialien zur Herstellung des Trägers können aus der Gruppe ausgewählt sein, die Polyester wie Polyethylenterephthalate (PET), Polycarbonate (PC), Polyetherketone (PAEK) Polyethylennaphthalat (PEN), Polybutylenterephthalate (PBT), Polyurethane (PU), Polystyrole (PS), Polyamide (PA), Polyoxymethylen (POM), Polyolefine wie Polyethylen (PE) und Polypropylen (PP), Polytetrafluorethylen (PTFE), Polyvinylchlorid (PVC), Polyvinylidenchlorid (PVDC), Polylactat (PLA) und Kunststoffe auf Cellulose-Basis wie Cellulosehydrat oder Celluloseacetat umfasst. Geeignete Materialien zur Herstellung des Trägers können auch aus der Gruppe der Metalle ausgewählt sein, die Aluminium, Eisen, Kupfer, Gold, Silber, Platin, Legierungen der vorgenannten Metalle und andere pharmazeutisch annehmbare Metallfolien oder mit Metall bedampfte Folien umfassen.

Vorzugsweise ist der Träger aus einem flexiblen Material hergestellt, beispielsweise einem Kunststoff. Ein Träger aus einem flexiblen Material kann sich besser der Hautoberfläche und ihrer Krümmung anpassen als ein Träger aus einem nicht flexiblen Material. Dadurch wird ein besserer Kontakt des Mikronadelarrays mit der Haut erreicht, wodurch die Verlässlichkeit des Mikronadelarrays verbessert wird.

In einer bevorzugten Ausführungsform kann der Träger ebenfalls mit einem Antiseptikum versehen sein.

Ein weiterer Gegenstand der vorliegenden Erfindung ist ein Verfahren zur Herstellung des erfindungsgemäßen Mikronadelarrays, das sich insbesondere dadurch auszeichnet, dass die Mikronadeln aus einem biologisch abbaubaren Polymer hergestellt sind, insbesondere aus diesem bestehen, bei dem ein Antiseptikum in die Polymermatrix eingebettet ist.

Das erfindungsgemäße Verfahren umfasst die folgenden Schritte:
a) Bereitstellen einer flüssigen Formulierung umfassend ein biologisch abbaubares Polymer, einen Wirkstoff und ein Antiseptikum mit mindestens einer Guanid- und/oder Biguanidgruppe, wobei das Antiseptikum und der Wirkstoff in die Polymermatrix der Mikronadeln integriert sind, und es sich bei dem Wirkstoff um einen Impfstoff handelt;
b) Einfüllen der Formulierung in eine flexible Form, die einem Negativabdruck des herzustellenden Mikronadelarrays entspricht;
c) Trocknen der Formulierung in der Form; und
d) Entfernen des getrockneten Mikronadelarrays aus der Form.

In einer bevorzugten Ausführungsform liegt das biologisch abbaubare Polymer und/oder das Antiseptikum in der bereitgestellten flüssigen Formulierung in Form seiner Monomere vor.

Das Trocknen der Formulierung in den flexiblen Formen unter Ausbildung des Mikronadelarrays kann auf dem Fachmann bekannte Weise erfolgen, vorzugsweise jedoch mittels Erwärmen und/oder im Luftzug.

Das Antiseptikum liegt in der Formulierung vorzugsweise in einer Menge vor, die einen Einbau in die Polymermatrix erlaubt und gleichzeitig eine ausreichende antiseptische Wirkung erreicht, um das Risiko einer Infektion durch die Mikronadeln zu minimieren. In einer bevorzugten Ausführungsform beträgt die Menge an Antiseptikum in der flüssigen Formulierung 100 bis 600 µg, vorzugsweise 150 bis 550 µg, insbesondere 200 µg oder 400 µg, jeweils bezogen auf 1 ml der flüssigen Formulierung.

In einer bevorzugten Ausführungsform können weitere Formulierungen in die flexible Form eingebracht werden, so dass ein mehrschichtiger Aufbau entsteht.

Ein weiterer Gegenstand der vorliegenden Erfindung ist die Verwendung einer Formulierung enthaltend ein biologisch abbaubares Polymer, einen Wirkstoff und ein Antiseptikum mit mindestens einer Guanid- und/oder Biguanidgruppe zur Herstellung von Mikronadelarrays, insbesondere Mikronadelarrays für die transdermale Applikation, wobei es sich bei dem Wirkstoff um einen Impfstoff handelt.

Die vorliegende Erfindung wird anhand der folgenden Beispiele weiter erläutert, wobei diese keinesfalls als Einschränkung des Erfindungsgedanken zu verstehen sind.

### Beispiel:

Die antibakterielle Wirkung des erfindungsgemäßen Mikronadelarray wurde mittels eines Hemmhoftests überprüft. Dazu wurde ein Mikronadelarray aus einer Formulierung, die Dextran als biologisch abbaubare Polymer und 200 µg/ml PHMB enthielt, hergestellt und auf eine Agarplatte mit Bakterien aufgebracht. Nach einer Inkubationszeit von 24 Stunden ist deutlich die Ausbildung eines Hemmhofs als Maß für die Minderung des Bakterienwachstums um das Mikronadelarray herum zu erkennen, wobei die Auflagestellen der Mikroarrays jeweils mit Hilfe von weißen Punkten gekennzeichnet sind. Überraschenderweise wurden die gleichen Ergebnisse auch mit Mikronadelarrays erzielt, die bereits einen Monat gelagert wurden.

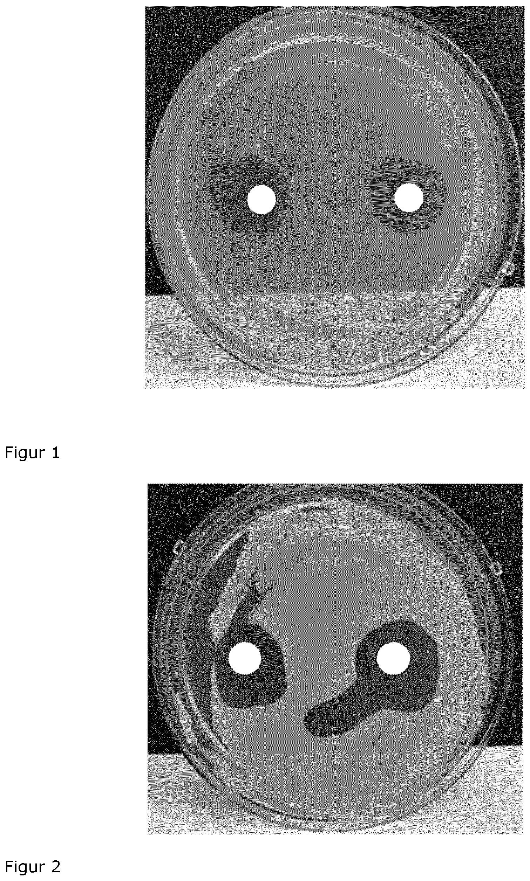

Figur 1 zeigt die antibakterielle Wirkung des erfindungsgemäßen Mikronadelarrays gegenüber *Pseudomonas aeruginosa.*

Figur 2 zeigt die antibakterielle Wirkung des erfindungsgemäßen Mikronadelarrays gegenüber *Staphylococcus aureus.*

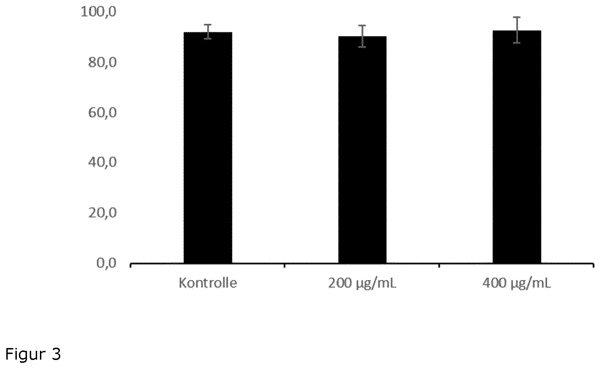

Des Weiteren wurde der Einfluss des Antiseptikums auf weitere in der Formulierung enthaltene Wirkstoffe getestet, wobei als Beispielswirkstoff das Hepatitis B S-Antigen (HBsAg) und als Antiseptikum PHMB verwendet wurde. Ausgehend von der Überlegung, dass, falls das Antiseptikum die Integrität des HBsAg beeinträchtigen sollte, sich dies in einer reduzierten Bindung des Wirkstoffs in einem HBsAg-spezifischen ELISA niederschlagen sollte, wurden erfindungsgemäße Mikronadelarrays aus einer Formulierung aus Dextran und 200 µg/ml beziehungsweise 400 µg/ml PHMB und 20 µm HBsAg pro Mikroarray hergestellt und vermessen. Als ELISA wurde ein HBsAg ELISA Kit von Alpha Diagnostic verwendet. Die Ergebnisse sind in Figur 3 zusammengefasst, die die beiden erfindungsgemäßen Mikroarrays mit jeweils 200 µg/ml und 400 µg/ml PHMB sowie die Kontrolltestreihe ohne PHMB zeigt. Je geringer die Wiederfindung im ELISA, desto größer der Einfluss des Antiseptikums auf den Wirkstoff. Wie den Daten der Figur 3 zu entnehmen ist, ist kein signifikanter Unterschied in der Antigenbindung zwischen der Kontrolle und den PHMB-haltigen Proben zu erkennen.

## Patentansprüche

1. Mikronadelarray zur Verwendung in der intradermalen Applikation, umfassend eine Vielzahl von Mikronadeln auf einem Träger, **dadurch gekennzeichnet, dass** die Mikronadeln aus einer Co-Formulierung gebildet sind, die ein biologisch abbaubares Polymer, einen Wirkstoff und ein Antiseptikum mit mindestens einer Guanid- und/oder Biguanidgruppe enthält, wobei das Antiseptikum und der Wirkstoff in die Polymermatrix der Mikronadeln integriert sind, und es sich bei dem Wirkstoff um einen Impfstoff handelt.

2. Mikronadelarray zur Verwendung gemäß Anspruch 1, **dadurch gekennzeichnet, dass** es sich bei dem Antiseptikum um ein Polybiguanid, vorzugsweise um Chlorhexidin und/oder Polyhexamethylenbiguanid (PHMB), insbesondere um Poly-hexamethylenbiguanid (PHMB) handelt.

3. Mikronadelarray zur Verwendung gemäß wenigstens einem der vorangehenden Ansprüche, **dadurch gekennzeichnet, dass** der Wirkstoff ausgewählt ist aus der Gruppe bestehend Nukleinsäuren, Peptiden und Proteinen.

4. Mikronadelarray zur Verwendung gemäß wenigstens einem der vorangehenden Ansprüche, **dadurch gekennzeichnet, dass** der Wirkstoff in Form von umhüllten Lipid-Nanopartikeln vorliegt.

5. Mikronadelarray zur Verwendung gemäß wenigstens einem der vorangehenden Ansprüche, **dadurch gekennzeichnet, dass** das biologisch abbaubare Polymer ausgewählt ist aus der Gruppe bestehend aus Polyvinylpyrrolidon, Polyvinylalkohole, Cellulose, Dextrane, Glycane, Glycosaminglycane, Hyaluronanen, alpha-Hydroxysäuren, solche wie Milchsäure und/oder Glykolsäure, Polylactide, Polyglykolide, Polylactid-co-Glykolide, und Copolymeren davon mit Polyethylenglykolen, Polyanhydriden, Poly(ortho)estern, Polyurethanen, Polybuttersäuren, Polyvaleriansäuren und Polylactid-co-Caprolactonen ausgewählt ist.

6. Verfahren zur Herstellung eines Mikronadelarrays zur Verwendung gemäß wenigstens einem der Ansprüche 1 bis 5, umfassend die Schritte:
a) Bereitstellen einer flüssigen Formulierung umfassend ein biologisch abbaubares Polymer, einen Wirkstoff und ein Antiseptikum mit mindestens einer Guanid- und/oder Biguanidgruppe;
b) Einfüllen der Formulierung in eine flexible Form, die einem Negativabdruck des herzustellenden Mikronadelarrays entspricht;
c) Trocknen der Formulierung in der Form; und
d) Entfernen des getrockneten Mikronadelarrays aus der Form.

7. Verfahren gemäß Anspruch 6, **dadurch gekennzeichnet, dass** die Menge an Antiseptikum in der flüssigen Formulierung 100 bis 600 µg, vorzugsweise 150 bis 550 µg, insbesondere 200 µg oder 400 µg beträgt, jeweils bezogen auf 1 ml der Formulierung.

8. Verwendung einer Formulierung enthaltend ein biologisch abbaubares Polymer, einen Wirkstoff und ein Antiseptikum mit mindestens einer Guanidund/oder Biguanidgruppe zur Herstellung von Mikronadelarrays, wobei es sich bei dem Wirkstoff um einen Impfstoff handelt.

## Claims

1. A microneedle array for use in intradermal administration, comprising a plurality of microneedles on a support, **characterized in that** the microneedles are formed from a co-formulation containing a biodegradable polymer, an active substance, and an antiseptic with at least one guanide and/or biguanide group; wherein the antiseptic and the active substance are integrated in the polymer matrix of the microneedles, and the active substance is a vaccine.

2. The microneedle array for use according to claim 1, **characterized in that** said antiseptic is a polybiguanide, preferably chlorhexidine and/or polyhexamethylenebiguanide (PHMB), especially polyhexamethylenebiguanide (PHMB).

3. The microneedle array for use according to at least one of the preceding claims, **characterized in that** said active substance is selected from the group consisting of nucleic acids, peptides and proteins.

4. The microneedle array for use according to at least one of the preceding claims, **characterized in that** said active substance is in the form of enveloped lipid nanoparticles.

5. The microneedle array for use according to at least one of the preceding claims, **characterized in that** said biodegradable polymer is selected from the group consisting of polyvinylpyrrolidone, polyvinyl alcohols, cellulose, dextrans, glycans, glycosaminoglycans, hyalurones, alpha-hydroxy acids, such as lactic acid and/or glycolic acid, polylactides, polyglykolides, poly(lactide-co-glykolide), and copolymers thereof with polyethylene glycols, polyanhydrides, poly(ortho)esters, polyurethanes, polybutyric acids, polyvaleric acids, and poly(lactide-co-caprolactone)s.

6. A process for preparing a microneedle array for use according to at least one of claims 1 to 5, comprising the steps of:
a) providing a liquid formulation comprising a biodegradable polymer, an active substance, and an antiseptic with at least one guanide and/or biguanide group;
b) filling the formulation into a flexible mold that corresponds to a negative print of the microneedle array to be prepared;
c) drying the formulation in the mold; and
d) demolding the dried microneedle array.

7. The process according to claim 6, **characterized in that** the amount of antiseptic in the liquid formulation is from 100 to 600 µg, preferably from 150 to 550 µg, especially 200 µg or 400 µg, respectively based on 1 ml of the formulation.

8. Use of a formulation containing a biodegradable polymer, an active substance, and an antiseptic with at least one guanide and/or biguanide group for preparing microneedle arrays, wherein the active substance is a vaccine.

## Revendications

1. Réseau de micro-aiguilles à utiliser dans l'administration intradermique, comprenant une pluralité de micro-aiguilles sur un support, **caractérisé en ce que** les micro-aiguilles sont formées d'une co-préparation contenant un polymère biodégradable, un principe actif et un antiseptique comportant au moins un groupe guanide et/ou biguanide, dans lequel l'antiseptique et le principe actif sont intégrés dans la matrice polymère des micro-aiguilles, et le principe actif est une vaccine.

2. Réseau de micro-aiguilles à utiliser selon la revendication 1, **caractérisé en ce que** l'antiseptique est un polybiguanide, de préférence chlorhexidine et/ou polyhexaméthylène biguanide (PHMB), notamment polyhexaméthylène biguanide (PHMB).

3. Réseau de micro-aiguilles à utiliser selon au moins l'une des revendications précédentes, **caractérisé en ce que** ledit principe actif est choisi dans le groupe consistant en acides nucléiques, peptides et protéines.

4. Réseau de micro-aiguilles à utiliser selon au moins l'une des revendications précédentes, **caractérisé en ce que** ledit principe actif est présent sous forme de nanoparticules lipidiques enrobées.

5. Réseau de micro-aiguilles à utiliser selon au moins l'une des revendications précédentes, **caractérisé en ce que** ledit polymère biodégradable est choisi dans le groupe consistant en polyvinylpyrrolidone, alcools polyvinyliques, cellulose, dextranes, glycanes, glycosaminoglycanes, hyaluronanes, acides alpha-hydroxylés - tels que l'acide lactique et/ou l'acide glycolique - polylactides, polyglycolides, polylactide-co-glycolides, et leurs copolymères avec des polyéthylène glycols, polyanhydrides, poly(ortho)esters, polyuréthanes, acides polybutyriques, acides polyvalériques et polylactide-co-caprolactones.

6. Procédé pour préparer un réseau de micro-aiguilles à utiliser selon au moins l'une des revendications 1 à 5, comprenant les étapes consistant à :
a) fournir une préparation liquide comprenant un polymère biodégradable, un principe actif et un antiseptique comportant au moins un groupe guanide et/ou biguanide ;
b) verser la préparation dans un moule flexible qui correspond à une empreinte négative du réseau de micro-aiguilles à préparer ;
c) sécher la préparation dans le moule ; et
d) démouler le réseau de micro-aiguilles séché.

7. Procédé selon la revendication 6, **caractérisé en ce que** la quantité de l'antiseptique dans la préparation liquide est de 100 à 600 µg, de préférence de 150 à 550 µg, notamment de 200 µg à 400 µg, dans chaque cas par rapport à 1 ml de la préparation.

8. Utilisation d'une préparation contenant un polymère biodégradable, un principe actif et un antiseptique comportant au moins un groupe guanide et/ou biguanide pour préparer des réseaux de micro-aiguilles, dans laquelle le principe actif est une vaccine.
